(19) Europäisches Patentamt
European Patent Office
Office européen des brevets

(11) Veröffentlichungsnummer: **0 683 159 A1**

# (12) EUROPÄISCHE PATENTANMELDUNG

(21) Anmeldenummer: **95106885.7**

(22) Anmeldetag: **06.05.95**

(51) Int. Cl.[6]: **C07D 271/08**, A61K 31/41, C07D 413/12, C07D 498/04

(30) Priorität: **20.05.94 DE 4417705**

(43) Veröffentlichungstag der Anmeldung: **22.11.95 Patentblatt 95/47**

(84) Benannte Vertragsstaaten: **AT BE CH DE ES FR GB IT LI NL SE**

(71) Anmelder: **CASSELLA Aktiengesellschaft**
**Hanauer Landstrasse 526**
**D-60386 Frankfurt (DE)**

(72) Erfinder: **Bohn, Helmut, Dr.**
**Kranzbergring 11**
**D-61137 Schöneck (DE)**
Erfinder: **Brendel, Joachim, Dr.**
**Landgrabenstrasse 23**
**D-61118 Bad Vilbel (DE)**
Erfinder: **Schönafinger, Karl, Dr.**
**Holunderweg 8**
**D-63755 Alzenau (DE)**
Erfinder: **Strobel, Hartmut, Dr.**
**Wasserloser Strasse 3**
**D-63755 Alzenau (DE)**

(74) Vertreter: **Muley, Ralf, Dr.**
**Cassella AG,**
**Patentabteilung,**
**Hanauer Landstrasse 526**
**D-60386 Frankfurt (DE)**

## (54) Substituierte Furoxane.

(57) Die vorliegende Erfindung betrifft Furoxane der allgemeinen Formel I

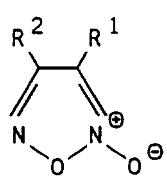

(I)

worin einer der Reste $R^1$ und $R^2$ für $-S(O)_n-R^3$ und der andere für $(C_1-C_{10})$-Alkyl, $(C_3-C_7)$-Cycloalkyl, $-CONR^4R^5$, -CN oder $-XR^6$ steht, Verfahren zu ihrer Herstellung und ihre Verwendung.

EP 0 683 159 A1

Die vorliegende Erfindung betrifft thio-, sulfinyl- oder sulfonyl-substituierte Furoxane, Verfahren zu ihrer Herstellung und ihre Verwendung.

Es sind bereits verschiedene thio-, sulfinyl- oder sulfonyl-substituierte Furoxane bekannt und beispielsweise beschrieben in J. Chem. Soc. 1964, 904; Synth. Comm. 1, 121 (1971); J. Heterocyclic Chem. 10, 587 (1973); Synth. Comm. 4, 311 (1974); Eur. J. Med. Chem. 1977, 157; J. Heterocyclic Chem. 14, 1415 (1977); Eur. J. Med. Chem. 1980, 485; J. Heterocyclic Chem. 19, 427 (1982); Tetrahedron 41, 727 (1985); Heterocycles 24, 889 (1986); Biochem. Pharm. 43, 1281 (1992); J. Med. Chem. 35, 3296 (1992); J. Chem. Soc. Perkin Trans. 2 1992, 1643; Il Farmaco 48, 321 (1993); EP-A 571 795 und WO 94/01422.

Die vorliegende Erfindung betrifft Furoxane der allgemeinen Formel I

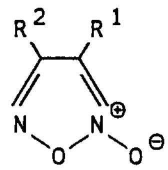

(I)

worin einer der Reste $R^1$ und $R^2$ für $-S(O)_n-R^3$ und der andere für $(C_1-C_{10})$-Alkyl, $(C_3-C_7)$-Cycloalkyl, $-CONR^4R^5$, -CN oder $-XR^6$ steht, wobei

| | |
|---|---|
| n | 0, 1 oder 2 bedeutet; |
| $R^3$ | $(C_1-C_{10})$-Alkyl, Hydroxy-$(C_1-C_{10})$-alkyl, $R^7R^8N-(C_1-C_{10})$-alkyl, $(C_2-C_{22})$-Alkyl, das durch ein, zwei oder drei Sauerstoffatome unterbrochen ist, $(C_3-C_7)$-Cycloalkyl, $(C_7-C_{10})$- Aralkyl, $-(CH_2)_mCOY$, Pyridylmethyl, $(C_6-C_{14})$-Aryl, 5- bis 14-gliedriges Heteroaryl, $(C_6-C_{14})$-Aryl oder 5- bis 14-gliedriges Heteroaryl, die ein oder mehrfach substituiert sind durch einen oder mehrere Gruppen aus der Reihe $(C_1-C_5)$-Alkyl, $(C_3-C_7)$-Cycloalkyl, Formyl, $(C_1-C_4)$-Alkylcarbonyl, Amino, $(C_1-C_5)$-Alkylamino, Di-$(C_1-C_5)$-alkylamino, Hydroxy, $(C_1-C_5)$-Alkoxy, Nitro, Cyano oder Halogen, oder $-CH_2CH_2SCOO(C_1-C_4)$-Alkyl bedeutet; |
| $R^4$ und $R^5$ | unabhängig voneinander Wasserstoff bedeuten oder wie $R^3$ definiert sind; |
| $R^6$ | unabhängig von diesem wie $R^3$ definiert ist, wobei $-CH_2CH_2SCOO(C_1-C_4)$-Alkyl ausgeschlossen ist und wobei, falls X für Schwefel steht, $R^6$ zusammen mit $R^3$ eine Ethylengruppe bilden kann; |
| $R^7$ und $R^8$ | unabhängig voneinander Wasserstoff, $(C_1-C_{10})$-Alkyl, $(C_7-C_{10})$-Aralkyl oder $(C_6-C_{14})$-Aryl, das wie in der Definition von $R^3$ angegeben substituiert sein kann, bedeuten; |
| X | Sauerstoff oder Schwefel bedeutet; |
| Y | Hydroxy, $(C_1-C_4)$-Alkoxy, Amino, $(C_1-C_4)$-Alkylamino oder Di-$(C_1-C_4)$-Alkylamino bedeutet; und |
| m | 1, 2 oder 3 bedeutet; |

sowie deren pharmakologisch annehmbare Salze, wobei, wenn einer der beiden Reste $R^1$ und $R^2$ für Methyl steht, $R^3$ nicht Phenyl oder durch Methyl, Methoxy, Chlor oder Fluor para-substituiertes Phenyl sein kann; wenn einer der beiden Reste $R^1$ und $R^2$ für Methyl steht und n = 0 oder 2 bedeutet, $R^3$ nicht Methyl oder Ethyl sein kann; wenn einer der beiden Reste $R^1$ und $R^2$ für Methyl steht und n = 2 bedeutet, $R^3$ nicht Benzyl sein kann; und wenn einer der beiden Reste $R^1$ und $R^2$ für Ethoxy steht, der andere nicht $-SO_2C_6H_5$ oder $-SO_2C_6H_4-CH_3$ sein kann.

Alkylgruppen können geradkettig oder verzweigt sein und sind beispielsweise Methyl, Ethyl, n-Propyl, i-Propyl, n-Butyl, sek.-Butyl, i-Butyl, tert.-Butyl, Pentyl, Hexyl, Heptyl, Octyl, Nonyl und Decyl. Bevorzugt haben Alkylgruppen 1 bis 4 Kohlenstoffatome und sind besonders bevorzugt Methyl, Ethyl, n-Propyl, i-Propyl, n-Butyl und tert. Butyl.

$(C_3-C_7)$-Cycloalkyl ist bevorzugt Cyclopentyl oder Cyclohexyl. Unter $(C_2-C_{22})$-Alkyl, das durch ein, zwei oder drei Sauerstoffatome unterbrochen ist werden beispielsweise Alkoxyalkylgruppen, wie Methoxymethyl, Methoxyethyl, Methoxypropyl, Ethoxymethyl, Ethoxyethyl, Ethoxypropyl, Propoxymethyl, Propoxyethyl und Propoxypropyl, aber auch Polyether, beispielsweise der Formel

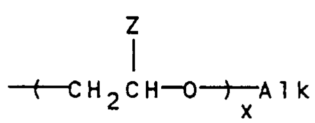

verstanden, worin

Z Wasserstoff oder Methyl,
x 1, 2 oder 3 und
Alk $(C_1-C_6)$-Alkyl

bedeuten.

$(C_7-C_{10})$-Aralkyl ist bevorzugt Benzyl oder Phenethyl. Pyridylmethyl ist beispielsweise Pyrid-2-yl-methyl, Pyrid-3-yl-methyl oder Pyrid-4-yl-methyl. $(C_6-C_{14})$-Aryl ist bevorzugt Phenyl. 5- bis 14-gliedriges Heteroaryl enthält als Heteroglieder insbesondere >O, >S,

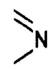

oder >$NR^9$,
worin $R^9$ wie $R^7$, aber unabhängig von diesem definiert ist. Sofern der Heterocyclus zwei Heteroglieder aufweist, können diese gleich oder verschieden sein. Ein Stickstoff enthaltender Heterocyclus kann auch über das Stickstoffatom gebunden sein und kann dann neben dem ersten, die Bindung vermittelnden Stickstoffatom noch ein beliebiges der oben genannten Heteroglieder enthalten.

Bevorzugtes Heteroaryl ist 5- oder 6-gliedrig, wobei sich besonders bevorzugte Reste ableiten von Pyrrol, Thiophen, Pyrazol, Imidazol, Oxazol, Isoxazol, Thiazol, Isothiazol, Pyridin, Pyridazin, Pyrimidin und Pyrazin. Die genannten Aryl- und Heteroarylgruppen können ein- oder mehrfach substituiert sein, wobei grundsätzlich jede geeignete Position einen Substituenten tragen kann.

Der Rest $-S(O)_nR^3$ bedeutet bevorzugt
$-S(O)_n-(C_1-C_4)$-Alkyl, $-S(O)_n$-Hydroxy-$(C_1-C_4)$-alkyl,
$-S(O)_n$-Cyclohexyl,
$-S(O)_nCH_2COY'$, worin Y' Hydroxy oder $(C_1-C_4)$-Alkoxy bedeutet,
$-S(O)_n$-Phenyl oder $-S(O)_n-CH_2CH_2SCOO(C_1-C_4)$-alkyl. Derjenige der Reste $R^1$ und $R^2$, der nicht für $-S(O)_nR^3$ steht, bedeutet bevorzugt $(C_1-C_4)$-Alkyl, $-CONHR^{4'}$, worin $R^{4'}$ Wasserstoff, $(C_1-C_4)$-Alkyl oder durch Halogen substituiertes Phenyl bedeutet, -CN, Cyclohexylthio, Phenylthio, $(C_1-C_4)$-Alkoxy, eine Gruppe der Formel

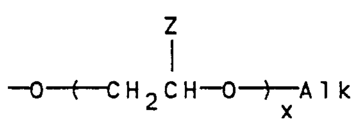

worin Z, x und Alk wie oben angegeben definiert sind, Pyrid-3-yl-methyloxy, $R^{7'}R^{8'}-N-(C_1-C_4)$-alkoxy, worin $R^{7'}$ und $R^{8'}$ unabhängig voneinander Methyl oder Benzyl bedeuten, oder $SCH_2COY'$, worin Y' wie oben angegeben definiert ist.

Die Verbindungen der allgemeinen Formel I können beispielsweise dadurch hergestellt werden, daß eine Verbindung der allgemeinen Formel II

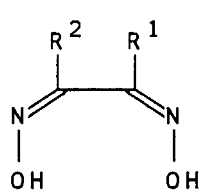

(II)

worin einer der Reste $R^1$ und $R^2$ für $-SR^3$ und der andere für $(C_1-C_{10})$-Alkyl, $(C_3-C_7)$-Cycloalkyl, $-CONR^4R^5$, -CN oder $-XR^6$ steht, wobei $R^3$, $R^4$, $R^5$, $R^6$ und X wie oben angegeben definiert sind, oxidiert wird und gegebenenfalls die so erhaltene Verbindung der allgemeinen Formel I, worin $n = 0$ ist, zur entsprechenden Verbindung der allgemeinen Formel I, worin $n = 1$ oder 2 ist, weiteroxidiert wird.

Zur Oxidation einer Verbindung der allgemeinen Formel II zu einer Verbindung der allgemeinen Formel I, worin $n = 0$ ist, können herkömmliche Reagentien wie zum Beispiel Halogene, N-Chlor- und N-Bromsuccinimid, Alkali- und Erdalkalihypochlorite, Alkylhypochlorite, wie z.B. tert. Butylhypochlorit, Blei-(IV)-Verbindungen, wie z.B. Bleitetraacetat, Eisen-(III)-Salze, wie z.B. rotes Blutlaugensalz, oder nitrose Gase, wie z.B. $N_2O_3$ oder $N_2O_4$ verwendet werden. Ein bevorzugtes Oxidationsmittel ist dabei $N_2O_4$.

Die Umsetzung wird bevorzugt in einem Lösungsmittel, wie beispielsweise Wasser, einem Alkohol, einem Ether, Essigester, Methylenchlorid, Cyclohexan, DMF, DMSO, Benzol, Toluol oder Chlorbenzol bei Temperaturen von -10°C bis 50°C, vorzugsweise von -5°C bis 25°C ausgeführt. Ein besonders bevorzugtes Lösungsmittel ist Diethylether.

Oxidationsmittel, die zur Umsetzung von Verbindungen der allgemeinen Formel I, in denen n = 0 ist, zu Verbindungen der allgemeinen Formel I, in denen n = 1 oder 2 ist, geeignet sind, sind beispielsweise Wasserstoffperoxid in essig- oder trifluoressigsaurem Medium, sowie Persäuren, vorzugsweise m-Chlorperbenzoesäure, die in einem organischen Lösungsmittel, insbesondere Methylenchlorid oder Aceton, eingesetzt werden. Die Temperatur liegt bei dieser Umsetzung bevorzugt bei -10 bis 50°C, besonders bevorzugt bei 20 bis 30°C.

Werden äquimolare Mengen Oxidationsmittel eingesetzt, so entstehen Verbindungen der allgemeinen Formel I, in denen n = 1 bedeutet, während die Verwendung überschüssigen Oxidationsmittels unter den gleichen Bedingungen, ohne die Notwendigkeit einer Isolierung der Zwischenstufe, zu Verbindungen der allgemeinen Formel I, in denen n = 2 bedeutet, führt.

Bei der Oxidation der Verbindungen der allgemeinen Formel II fallen in der Regel die Verbindungen der allgemeinen Formel I in Form von Isomerengemischen an. Diese lassen sich aber durch bekannte Methoden wie Umkristallisieren oder chromatographische Methoden, insbesondere Säulenchromatographie, trennen. Isomerengemische werden auch erhalten, wenn ein reines Isomeres in Substanz oder in einem inerten Lösungsmittel gelöst auf Temperaturen von 50 bis 200°C erhitzt oder bei 0 bis 50°C photolysiert wird. Durch Trennung des so erhaltenen Gemisches ist es somit möglich, ein Isomeres in das andere umzuwandeln.

Die Verbindungen der allgemeinen Formel II sind literaturbekannt oder können in Analogie zu bekannten Verfahren hergestellt werden.
Beispielsweise kann nach folgendem Schema vorgegangen werden:

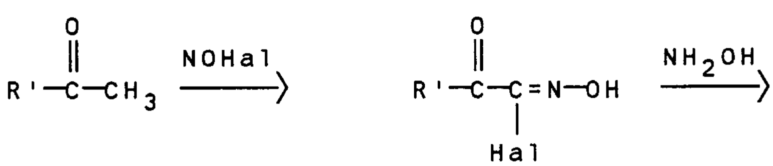


(III) (IV)

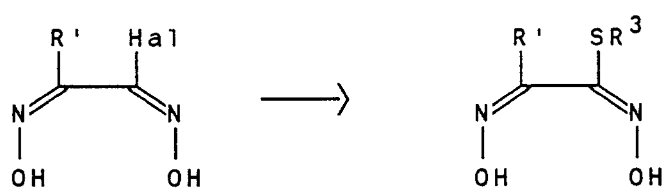

(V) (IIa)

In den allgemeinen Formeln III bis IIa bedeuten dabei R' ($C_1$-$C_{10}$)-Alkyl, ($C_3$-$C_7$)-Cycloalkyl, -$CONR^4R^5$, -CN oder $XR^6$, wobei $R^3$, $R^4$, $R^5$ und $R^6$ wie oben angegeben definiert sind, und Hal Halogen, insbesondere Chlor.

Die Umsetzung der Verbindung der allgemeinen Formel III mit z.B. Nitrosylchlorid wird beispielsweise in Tetrachlorkohlenstoff oder, bevorzugt, in Ether bei 0 bis 20°C ausgeführt. Die anschließende Oximierung mit Hydroxylamin erfolgt in alkoholischer oder bevorzugt wäßriger Lösung (Liebigs Ann. Chem. 44, 113 (1925), Tetrahedron 19, 143 (1963)).

Die Hydroximinoylhalogenide der allgemeinen Formel V werden anschließend mit einem Thiol $HSR^3$ zur Verbindung der Formel IIa umgesetzt. Bevorzugt wird dabei die Verbindung der allgemeinen Formel V in einem organischen Lösungsmittel, beispielsweise einem niederen Alkohol, Ether oder Ester, bevorzugt aber in Diethylether, vorgelegt, eine Hilfsbase, beispielsweise Alkoholat, anorganisches Carbonat oder Hydroxid oder ein tertiäres Amin, bevorzugt Triethylamin, in 10 bis 15 mol%igem Überschuß zugegeben und das

Thiol in 5 bis 10 mol%igem Überschuß zugetropft. Die Temperatur liegt dabei bevorzugt bei 0 bis 60°C, besonders bevorzugt 20 bis 40°C.

In einer alternativen Vorgehensweise kann die Verbindung der allgemeinen Formel IV auch zuerst mit dem Thiol $HSR^3$ und anschließend mit Hydroxylamin umgesetzt werden, wobei die oben angegebenen Reaktionsbedingungen auch in diesem Falle anzuwenden sind.

Die Verbindungen der allgemeinen Formel V können darüberhinaus auch nach folgendem Schema erhalten werden:

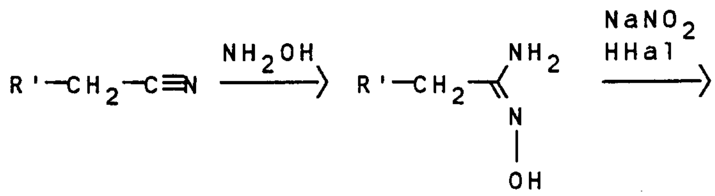


(VI) (VII) (V)

Die Nitrile der allgemeinen Formel VI können dabei in die Amidoxime der allgemeinen Formel VII überführt werden (Chem. Heterocycl. Compd. 1985, 988), deren Nitrosierung in 10 - 60%iger Mineralsäure HHal zu den Verbindungen der allgemeinen Formel V führt (Synth. Commun. 22, 453 (1992)). Sollen Verbindungen der allgemeinen Formel I, in denen einer der beiden Reste $R^1$ und $R^2$ für $-CONR^4R^5$ steht, hergestellt werden, so können die entsprechenden Nitrile der allgemeinen Formel VIa

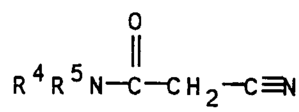

durch Aminierung von Cyanessigsäureestern erhalten werden (Aust. J. Chem. 29, 1039 (1976)).

Erfindungsgemäße Verbindungen der allgemeinen Formel I, in denen einer der Reste $R^1$ und $R^2$ für $CONH_2$ steht, können durch alkalische Verseifung entsprechender Nitrile erhalten werden. Diese Umsetzung wird bevorzugt in einem Lösungsmittel, insbesondere Wasser, unter Zusatz von Alkalimetallhydroxiden und Zugabe von Wasserstoffperoxid durchgeführt. Die Temperatur liegt dabei bevorzugt bei 0 bis 50°C.

Erfindungsgemäße Verbindungen der allgemeinen Formel Ia

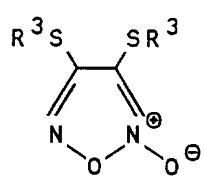

(Ia)

worin $R^3$ wie oben angegeben definiert ist, können durch basenkatalysierte Umsetzung von Dichlorglyoxim mit Thiolen $R^3SH$ oder Thioglykol und nachfolgender Oxidation erhalten werden.

Bei der Umsetzung mit den Thiolen $R^3SH$ werden 2 Moläquivalente pro Mol Dichlorglyoxim verwendet, bei der Umset zung mit Thioglykol werden die Reaktionspartner im Molverhältnis 1:1 eingesetzt.

Die Reaktion wird im allgemeinen in Gegenwart einer Base, vorzugsweise in stöchiometrischen Mengen, durchgeführt, wobei Alkalimetallalkoholate, wie Natriummethanolat, Alkalimetallhydroxide oder -carbonate, wie Kaliumcarbonat oder Trialkylamine, wie Triethylamin, besonders bevorzugt sind.

Die Verbindungen der allgemeinen Formel Ia können durch Oxidation zu den entsprechenden Bissulfonylfuroxanen und anschließenden sequentiellen Austausch der Sulfonylreste zu erfindungsgemäßen Verbindungen der allgemeinen Formel Ib

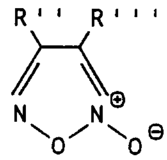

(Ib)

worin R'' $-XR^6$ und R''' $-SO_2R^3$ oder $-SR^3$ bedeuten, umgesetzt werden. Dazu werden die genannten Bissulfonylfuroxane, die wie oben angegeben oder nach sonstigen literaturbekannten Verfahren (z.B. J.Heterocyclic Chem. 14, 1415 (1977)) hergestellt sein können, in einem Lösungsmittel, beispielsweise einem Alkohol, Wasser oder Aceton, gelöst und unter Zusatz einer Base, beispielsweise Natriumhydroxid, Natriumalkoholat oder einem Alkalicarbonat, mit einem Alkohol $HOR^6$ oder einem Mercaptan $HSR^6$ umgesetzt. Hierbei wird selektiv nur die 4-Sulfongruppe ausgetauscht. Gewünschtenfalls kann anschließend auch die 3-Sulfonylgruppe durch erneute Umsetzung mit einem Mercaptan $HSR^6$ ausgetauscht werden.

Erfindungsgemäße Verbindungen der allgemeinen Formel I, die eine basische Gruppe enthalten, können mit anorganischen oder organischen Säuren Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden. Erfindungsgemäße Verbindungen, die eine saure Gruppe enthalten, können mit anorganischen oder organischen Basen Salze bilden, beispielsweise Natrium- oder Kaliumsalze.

Die Verbindungen der allgemeinen Formel I und ihre pharmakologisch annehmbaren Salze besitzen wertvolle pharmakologische Eigenschaften. Am Modell der Kalium-depolarisierten Pulmonalarterie des Meerschweinchen (Godfraind and Kaba (Arch.Int.Pharmacodyn.Ther. 196, (Suppl) 35 bis 49, 1972) führen sie in niedrigen Konzentrationen zu einer lang anhaltenden Relaxation. Diese Wirkung kann mit Oxyhämoglobin inhibiert werden, was auf einen NO-mediierten Mechanismus deutet. Stickstoffmonoxid führt als Aktivator der Guanylatcyclase zu einer Erhöhung von cyclischem Guanosinmonophosphat, welches im glatten Muskel eine Relaxation und in den Blutplättchen antiadhäsive und antiaggregatorische Wirkungen verursacht. Stickstoffmonoxid ist außerdem auch entscheidend beteiligt bei Lernvorgängen, bei der Regulation der Nierenfunktion, bei der Immunabwehr, beim septischen Schock und bei erektilen Dysfunktionen. Die erfindungsgemäßen Verbindungen können somit bei den genannten Indikationen eingesetzt werden.

Vor allem aber haben sich NO-Donoren zur Behandlung und Prophylaxe von angina pectoris bewährt.

Die Verbindungen der allgemeinen Formel I und ihre pharmakologisch annehmbaren Salze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger-und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben, elastischen Flüssigpflastern, transdermalen Systemen oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen

Drucks, Überzugsmittel oder Antioxidantien enthalten. Sie können auch zwei oder mehrere Verbindungen der allgemeinen Formel I oder ihrer pharmakologisch annehmbaren Salze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der allgemeinen Formel I, ihre pharmakologisch annehmbaren Salze und pharmazeutische Präparate, welche die Verbindungen der allgemeinen Formel I oder ihre pharmakologisch annehmbaren Salze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Darüberhinaus können sie auch zur Behandlung erektiler Dysfunktionen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z. B. 2 bis 4 Teilverabreichungen aufgeteilt.

## Beispiel 1

## N-Isopropyl-3-phenylmercapto-furoxan-4-carbonamid

**a)** 3-Amino-3-hydroximino-N-isopropylpropionamid

Eine Lösung von 93,6 g (0,9 mol) Natriumcarbonat in 240 ml Wasser wird mit einer Lösung von 61,3 g (0,9 mol) Hydroxylaminhydrochlorid versetzt, eine halbe Stunde gerührt und mit 100 ml Ethanol versetzt. Die so erhaltene Lösung wird zu einer Lösung von 87 g (0,7 mol) Cyano-N-isopropylacetamid gegeben und 1h auf 70°C erhitzt. Nach Stehen über Nacht wird vom Niederschlag abgesaugt, eingeengt, der Rückstand mit 200 ml Wasser verrieben, erneut abgesaugt und der verbleibende Rückstand aus Ethanol umkristallisiert.

Man erhält 69,9 g (63%) 3-Amino-3-hydroximino-N-isopropylpropionamid

Fp.: 135°C

**b)** 3-Chlor-2,3-bis-hydroximino-N-isopropylpropionamid

115 g (0,72 mol) 3-Amino-3-hydroximino-N-isopropylpropionamid werden in 1320 ml konz. HCl gelöst, auf 0°C gekühlt und eine Lösung von 149 g (2,16 mol) Natriumnitrit in 360 ml Wasser so zugetropft, daß die Temperatur 5°C nicht überschreitet. Zur Aufarbeitung wird abgesaugt, in 2000 ml Essigester suspendiert, mit 500 ml $H_2O$ ausgerührt und die organische Phase nach Trocknen über $Na_2SO_4$ eingeengt.

Man erhält 28 g (18%) 3-Chlor-2,3-bis-hydroximino-N-isopropylpropionamid.

Das Produkt schmilzt unter Zersetzung

$^1$H-NMR: 1,04 (d, 6H, $CH_3$), 3,94 (oct, 1H, CH-N), 8,17 (d, 1H, NH), 12,08 (s, 1H, OH), 12,80 (s, 1H, OH)

**c)** 2,3-Bis-hydroximino-N-isopropyl-3-phenylmercapto-propionamid

22,8 g (0,11 mol) 3-Chlor-2,3-bishydroximino-N-isopropylpropionamid und 13,3 g (0,12 mol) Thiophenol werden in 1000 ml Diethylether vorgelegt und eine Lösung von 12,6 g (0,12 mol) Triethylamin in 100 ml Diethylether zugetropft.

Nach 1h Rühren bei RT wird nacheinander mit 1N $H_2SO_4$, gesättigter Natriumcarbonat-Lösung und Wasser gewaschen, mit $MgSO_4$ getrocknet und eingeengt, der Rückstand mit Hexan/Diethylether verrieben und abgesaugt.

Man erhält 21,1 g (68%) 2,3-Bis-hydroximino-N-isopropyl-3-phenylmercapto-propionamid

Fp.: 148,5°C

**d)** N-Isopropyl-3-phenylmercapto-furoxan-4-carbonamid

21 g (75 mmol) 2,3-Bis-hydroximino-N-isopropyl-3-phenylmercapto-propionamid werden in 500 ml Diethylether suspendiert und 8,24 g (90 mmol) Distickstofftetroxid bei 0°C zugetropft.

Nach 1h bei 0°C wird auf Eis-Wasser gegeben, die etherische Phase abgetrennt, getrocknet, eingeengt und der Rückstand aus Hexan/Ether 1:1 umkristallisiert.

Man erhält 14,6 g (70%) N-Isopropyl-3-phenylmercaptofuroxan-4-carbonamid
Fp.: 81°C

**Beispiel 2**

**4-Phenylmercapto-furoxan-3-carbonitril**

12,6 g (57 mmol) 2,3-Bishydroximino-3-phenylmercapto-propionitril werden in 600 ml Diethylether vorgelegt und bei 0°C 6,3 g (68 mmol) Distickstofftetroxid zugetropft. Nach 3h bei 0°C wird auf Eis-Wasser gegeben, die etherische Phase abgetrennt, getrocknet und eingeengt. Der Rückstand wird an Kieselgel mit Essigester : Hexan = 1:1 chromatographiert und die Hauptfraktion aus Ether/Hexan = 1:1 umkristallisiert.
Man erhält 6,12 g (49%) 4-Phenylmercapto-furoxan-3-carbonitril
Fp.: 59,5°C

**Beispiel 3**

**4-Phenylsulfinyl-furoxan-3-carbonitril und 4-Phenylsulfonyl-furoxan-3-carbonitril**

2 g (9,7 mmol) 4-Phenylmercaptofuroxan-3-carbonitril werden in 20 ml Aceton gelöst und eine zuvor mit 6 g $Na_2SO_4$ getrocknete Lösung von 7 g (~ 32,5 mmol) ca. 80%iger m-Chlorperbenzoesäure so weit zugetropft bis unter stetiger DC-Kontrolle der gewünschte Umsetzungsgrad eingestellt ist.
Zur Aufarbeitung wird auf Eis-Wasser gegossen, mit Methylenchlorid extrahiert und die organische Phase mehrfach mit Bicarbonat-Lösung, Natriumsulfitlösung und Wasser gewaschen. Der nach Trocknen und Einrotieren verbleibende Rückstand wird an Kieselgel mit $CH_2Cl_2$ : Hexan = 1:1 chromatographiert und die jeweilige Hauptfraktion aus Ether : Hexan umkristallisiert.
4-Phenylsulfinylfuroxan-3-carbonitril Fp.: 81°C
4-Phenylsulfonylfuroxan-3-carbonitril Fp.: 82°C

**Beispiel 4**

**4-Phenylmercaptofuroxan-3-carbonamid**

1,12 g (5,1 mmol) 4-Phenylmercaptofuroxan-3-carbonitril werden in 15 ml 2N NaOH suspendiert und 1,81 g (5,25 mmol) 10%ige Wasserstoffperoxidlösung zugetropft. Nach 4h bei RT wird abgesaugt und aus Isopropanol umkristallisiert.
Man erhält 0,7 g (58%) 4-Phenylmercaptofuroxan-3-carbonamid
Fp.: 139 - 140°C

**Beispiel 5**

**4-(β-(Methoxycarbonylmercapto)-ethylmercapto)-3-methylfuroxan**

**a)** 1-(2-(Methoxycarbonylmercapto)-ethylmercapto)-2-methyl-glyoxim
20,0 g (0,15 mol) 1-Chlor-2-methyl-glyoxim werden zusammen mit 24,5 g (0,16 mol) β-Mercaptoethylthiokohlensäuremethylester in 1500 ml Diethylether und 16,75 g (0,16 mol) Triethylamin in 60 ml Ether zugetropft. Es wird 18h bei RT gerührt und 2h unter Rückfluß erhitzt.
Zur Aufarbeitung wird abgesaugt, die Ether-Phase mit Wasser gewaschen, getrocknet, eingeengt und aus Isopropanol umkristallisiert.
Man erhält 12,3 g (33%) 1-(2-Methoxycarbonylmercapto)-ethylmercapto)-2-methyl-glyoxim
Fp.: 147 - 151°C
**b) 4-(β-(Methoxycarbonylmercapto)-ethylmercapto)-3-methylfuroxan**
12,3 g (48,8 mmol) β-Methoxycarbonylmercapto-ethylmercapto-methyl-glyoxim werden in 340 ml Diethylether gelöst und bei 0°C 5,4 g (58,5 mmol) Distickstofftetroxid zugetropft. Nach 18h bei RT wird eingeengt, in Methylenchlorid aufgenommen, mit $H_2O$ gewaschen, getrocknet, eingeengt und der Rückstand an Kieselgel mit Hexan : Essigester = 3:2 chromatographiert.
Man erhält 9,9 g (81%) 4-(β-(Methoxycarbonylmercapto)-ethylmercapto)-3-methyl-furoxan als farbloses, viskoses Öl.
$^1$H-NMR: 2,08 (s, 3H, $CH_3$), 3,25 (t, 2H, α-$CH_2$),3,45 (t, 2H, β-$CH_2$), 3,80 (s, 3H, $CH_2O$)

**Beispiel 6**

**3-Methoxycarbonylmethylmercapto-4-methyl-furoxan**

3,8 g (18,6 mmol) 4-Methoxycarbonylmethylmercapto-3-methyl-furoxan (Beispiel 9) werden unter $N_2$ in 50 ml Xylol 4h auf 140°C erhitzt. Zur Aufarbeitung wird eingeengt und an Kieselgel mit Hexan : Essigester 3:1 chromatographiert.
Man erhält 0,85 g (22%) 3-Methoxycarbonylmethylmercapto-4-methyl-furoxan
Öl, $^1$H-NMR: 2,38 (s, 3H, $CH_3$), 3,62 (s, 3H, $CH_3O$), 4,82 (s, 2H, $CH_2$)
Analog den vorstehenden vorschriften können hergestellt werden:

**Beispiel 7**

**4-($\beta$-Hydroxyethylmercapto)-3-methylfuroxan**

Öl, $^1$H-NMR: 2,08 (s, 1H, $CH_3$) 3,27 (t, 2H, H-$\alpha$) 3,72 (q, 2H, H-$\beta$) 5,20 (t, 1H, OH)
Vorstufe: 1-($\beta$-Hydroxyethylmercapto)-2-methyl-glyoxim Fp.: 129°C

**Beispiel 8**

**4-(($\beta$-Methoxycarbonylmercapto)-ethylsulfinyl)-3-methylfuroxan**

Öl, $^1$H-NMR: 2,24 (s, 1H, $CH_3$) 3,25-3,40 (m, 2H, H-$\beta$) 3,55-3,75 (m, 2H-H-$\alpha$) 3,80 (s, 3H, $CH_3O$)

**Beispiel 9**

**4-Methoxycarbonylmethylmercapto-3-methyl-furoxan**

Fp.: 49°C
Vorstufe: 1-Methoxycarbonylmethylmercapto-2-methylglyoxim
Fp.: 121 - 126°C (d)
$^1$H-NMR: 1,95 (s, 3H, $CH_3$) 3,61 (s, 3H, $OCH_3$) 3,88 (s, 2H, $CH_2$) 11,58 (s, 1H, OH) 12,01 (s, 1H, OH)

**Beispiel 10**

**4-Methoxycarbonylmethylsulfinyl-3-methyl-furoxan**

Öl, $^1$H-NMR: 2,13 (s, 3H, $CH_3$) 3,77 (s, 3H, $CH_3O$) 4,59 (AB-d, 2H, $CH_2$)

**Beispiel 11**

**S-(3-Methylfuroxan-4-yl)-mercaptoessigsäure**

Fp.: 106 - 108°C

**Beispiel 12**

**3-Phenylmercaptofuroxan-4-p-chlorcarbonanilid**

Fp.: 159 - 160°C
Vorstufe:
2,3-Bishydroximino-3-phenylmercapto-p-chlorpropionanilid
Fp.: 148°C

**Beispiel 13**

**4-Butylmercapto-furoxan-3-carbonamid**

Fp.: 95°C

**Beispiel 14**

**3-((β-Methoxycarbonylmercapto)-ethylmercapto)-furoxan-4-p-chlorcarbonanilid**

Fp.: 97 - 100°C

**Beispiel 15**

**3-β-Hydroxyethylmercapto-N-isopropyl-furoxan-4-carbonamid**

Öl, $^1$H-NMR: 1,20 (d, 6H, $CH_3$) 3,12 - 3,20 und 3,55-3,65 (m, 4H, $CH_2$), 4,10 (oct, 1H, CH-N), 5,05 (bs, 1H, OH), 9,00 (d, 1H, NH)
Vorstufe:
3-(β-Hydroxyethylmercapto)-2,3-bishydroximino-N-isopropylpropionamid
Öl, $^1$H-NMR: 1,10 (d, 6H, H iprop-$CH_3$), 3,11 (t, 2H, H-α) 3,52 (q, 2H, H-β), 3,90 (oct, 1H, i-propCH), 8,10 (d, 1H, NH), 11,79 (s, 1H, OH) 12,17 (s, 1H, OH)

**Beispiel 16**

**3-Butylmercapto-N-isopropyl-furoxan-4-carbonamid**

Öl, $^1$H-NMR: 0,85 (t, 3H, Butyl-4), 1,16 (d, 6H, iprop$CH_3$) 1,35 (sext, 2H, Butyl-3), 1,46 (quint., 2H, Butyl-2), 3,04 (t, 2H, Butyl-1), 4,06 (oct, 1H, iprop-CH), 9,03 (d, 1H, NH)
Vorstufe:
3-Butylmercapto-2,3-bishydroximino-N-isopropyl-propionamid
Öl, $^1$H-NMR: 0,89 (t, 3H, Butyl-4), 1,08 (d, 6H, iprop$CH_3$), 1,37 (sext, 2H, Butyl-3), 1,46 (quart, 2H, Butyl-2), 3,05 (t, 2H, Butyl-1), 3,95 (okt, 1H, iprop-CH), 8,12 (d, 1H, NH), 11,55 (s, 1H, OH), 12,26 (s, 1H, OH)

**Beispiel 17**

**4-Butylmercapto-furoxan-3-carbonitril**

Öl, $^1$H-NMR: 0,91 (t, 3H, Butyl-4), 1,43 (sext. 2H, Butyl-3), 1,73 (quint, 2H, Butyl-2), 3.25 (t, 2H, Butyl-1)

**Beispiel 18**

**3-Butylmercapto-furoxan-4-carbonitril**

Öl, $^1$H-NMR: 0,88 (t, 3H, Butyl-4) 1,35 (sext, 2H, Butyl-3), 1,58 (q, 2H, Butyl-2), 3,04 (t, 2H, Butyl-1)

**Beispiel 19**

**3-Butylsulfonyl-furoxan-4-carbonitril**

Öl, $^1$H-NMR: 0,90 (t, 3H, Butyl-4), 1,45 (sext., 2H, Butyl-3), 1,75 (quint, 2H, Butyl-2), 3,73 - 3,81 (m, 2H, Butyl-1)

**Beispiel 20**

**1,2,5-Oxadiazolo[3,4-b][1,4]dithian-N-oxid**

**a)** Zu einer Lösung von 9,4 g (60 mmol) Dichlorglyoxim (Tetrahedron 19 (1963) 143) und 5,65 g (60 mmol) Dithioglykol in 120 ml Methanol werden bei -40°C 21,6 g (120 mmol) einer 30%igen Natriummethanolatlösung langsam zugetropft. Man läßt innerhalb von 3h auf Raumtemperatur erwärmen, rührt noch 2h nach und engt dann komplett ein.
Man verrührt den Rückstand mit 40 ml Wasser, saugt ab und erhält 9,3 g (87%) 2,3-Bishydroxyimino-1,4-dithian.
Fp.: 202 - 205°C
**b)** Zu einer Lösung von 9,3 g 2,3-Bishydroxyimino-1,4-dithian in 330 ml 10%iger Natronlauge werden bei 0°C 330 ml 14% Natriumhypochloritlösung zugetropft. Der ausgefallene Niederschlag wird sofort abgesaugt, mit Wasser gewaschen und im Vakuum getrocknet. Man erhält 6,5 g (71%) [1,2,5]-Oxadiazolo[3,4-b][1,4]dithian-N-oxid,
Fp.: 78 - 79°C

**Beispiel 21**

**3,4-Biscyclohexylmercapto-furoxan**

**a)** Zu einer Lösung von 11,8 g (75 mmol) Dichlorglyoxim und 15 g (150 mmol) Cyclohexylmercaptan in 150 ml Methanol werden bei -40°C 27 g (150 mmol) einer 30%igen Natriummethanolatlösung langsam zugetropft. Man läßt innerhalb von 3h auf Raumtemperatur erwärmen, rührt über Nacht und saugt den gebildeten Niederschlag ab. Das Filtrat wird im Vakuum eingeengt, der Rückstand in 60 ml heißem Isopropanol gelöst und das Produkt durch Zugabe von 500 ml Petrolether ausgefällt. Man erhält 15,2 g (64%) Biscyclohexylmercaptoglyoxim.
Fp.: 160 - 161°C
**b)** Zu einer Lösung von 6,3 g Biscyclohexylmercaptoglyoxim in 125 ml 10%iger Natronlauge tropft man bei 0°C 125 ml 14% Natriumhypochloritlösung zu. Das ölig ausgefallene Produkt wird mit Essigester extrahiert und durch Chromatographie mit Cyclohexan/Essigester 90 : 5 gereinigt. Nach anschließender Umkristallisation aus Isopropanol erhält man 1,5 g 3,4-Biscyclohexylmercapto-furoxan.
Fp.: 80 - 81°C

**Beispiel 22**

**3,4-Bisphenylmercapto-furoxan**

Aus Dichlorglyoxim und Thiophenol wird analog Beispiel 21 3,4-Bisphenylmercapto-furoxan erhalten als leicht gelbliches Öl, das auch nach chromatographischer Reinigung nicht kristalliert. Als Strukturbeweis wird das Produkt durch Oxidation mit Wasserstoffperoxid in Eisessig in das bekannte 3,4-Bisphenylsulfonyl-1,2,5-oxa-diazol-2-oxid,
Fp.: 155°C, überführt.

**Beispiel 23**

**4-(2-Methoxyethyloxy)-3-phenylsulfonylfuroxan**

Eine Lösung von 6,0 g 3,4-Diphenylsulfonylfuroxan in 70 ml Aceton wird mit einer Lösung von 3,7 g Glykolmonomethylether in 26 ml Aceton sowie 1,0 g Natriumhydroxid in 8 ml Wasser versetzt, wobei sich die Reaktionsmischung auf 40°C erwärmt. Nach 1h kühlt man auf 10°C ab, filtriert vom ausgefallenen Natriumphenylsulfinat ab und engt das Filtrat ein. Der Rückstand wird mit 500 ml Essigester verrührt und erneut abfiltriert. Das Filtrat wird eingeengt, aus 30 ml Ethanol umkristallisiert, und man erhält 3,4 g (68%) 4-(2-Methoxyethyloxy)-3-phenylsulfonylfuroxan.
Fp.: 105 -107°C

**Beispiel 24**

**4-Methoxy-3-phenylsulfonylfuroxan**

Analog Beispiel 23 wurde 4-Methoxy-3-phenylsulfonylfuroxan in 80% Ausbeute erhalten.
Fp.: 109 - 111°C
$^{13}C$-NMR: $\delta$ = 58.3, 110.7, 128.3, 130.0, 136.1, 137.2, 159.6 ppm.

Das Signal bei 159.6 ppm, das von C-4 des Furoxanrings herrührt, weist eine 3,8 Hz-Kopplung zur Methoxygruppe auf, während das Signal bei 110.7 ppm, das von C-3 herrührt, nicht aufgespalten ist. Dies beweist die angegebene Substitution in 4-Position.

**Beispiel 25**

**4-(3-Pyridylmethyloxy)-3-phenylsulfonylfuroxan**

Zu einer Lösung von 30 g 3,4-Diphenylsulfonylfuroxan in 360 ml Aceton werden nacheinander 26,7 g 3-Hydroxymethylpyridin gelöst in 100 ml Aceton und 4,9 g Natriumhydroxid gelöst in 40 ml Wasser zugegeben. Nach 30 min wird das ausgefallene Natriumphenylsulfinat abgesaugt, und das Filtrat wird eingeengt. Der Rückstand wird durch Chromatographie über Kieselgel (Laufmittel : Essigester) gefolgt von Umkristallisation aus Ethanol gereinigt.
Man erhält 20,2 g (74%) 4-(3-Pyridylmethyloxy)-3-phenylsulfonylfuroxan
Fp.: 121°C

**Beispiel 26**

**4-(Methoxy-tri(ethylenoxy))-3-phenylsulfonylfuroxan**

Analog Beispiel 25 wurden aus 6,0 g 3,4-Diphenylsulfonylfuroxan, 5,8 g Triethylenglykolmonomethylether und 1,0 g Natriumhydroxid 4,3 g (68%) 4-(Methoxy-tri(ethylenoxy)-3-phenylsulfonylfuroxan erhalten, das nicht zur Kistallisation gebracht werden konnte.

| Elementaranalyse: | | | | | |
|---|---|---|---|---|---|
| gefunden: | 46,4% C, | 5,4% H, | 7,1% N, | 32,5% O, | 9,1% S |
| berechnet: | 46,4% C, | 5,2% H, | 7,2% N, | 33,0% O, | 8,3% S |

**Beispiel 27**

**4-[2-(N-Benzyl-N-methylamino)-ethyloxy]-3-phenylsulfonylfuroxanhydrochlorid**

10,0 g 3,4-Diphenylsulfonylfuroxan, 13,5 g N-Benzyl-N-methylethanolamin und 2,0 g Natriumhydroxid werden analog Beispiel 25 umgesetzt. Das Rohprodukt wird mit $CH_2Cl_2$/MeOH 99:1 gesäult und anschließend mit etherischer Salzsäure in das Hydrochlorid überführt.
Man erhält 2,4 g (20%) 4-[2-(N-Benzyl-N-methylamino)-ethyloxy]-3-phenylsulfonyl-furoxanhydrochlorid
Fp.: 171°C

**Beispiel 28**

**4-[2-(N,N-Dimethylamino)ethyloxy]-3-phenylsulfonylfuroxanhydrochlorid**

Die Herstellung erfolgte analog Beispiel 27.
Fp.: 142 - 144°C

**Beispiel 29**

**4-Methoxycarbonylmethylmercapto-3-phenylsulfonylfuroxan**

Eine Lösung von 2,0 g (5,5 mmol) 3,4-Diphenylsulfonylfuroxan in 10 ml Aceton wird mit 20 ml Methanol, 0,6 g (5,7 mmol) Thioglykolsäuremethylester und 0,6 g (5,9 mmol) Triethylamin versetzt und 2h unter Stickstoff gerührt. Man gießt die Reaktionsmischung auf 50 ml Wasser, saugt das ausgefallene Produkt ab und reinigt es durch Chromatographie.
Man erhält 0,75 g (42%) 4-Methoxycarbonylmethylmercapto-3-phenylsulfonylfuroxan.
Fp.: 118 - 120°C

**Beispiel 30**

**3-Butylmercapto-4-(3-pyridylmethyloxy)-furoxanhydrochlorid**

Eine Lösung von 5,0 g 4-(3-Pyridylmethyloxy)-3-phenylsulfonylfuroxan (Beispiel 25) und 1,6 g Butylmercaptan in 500 ml Methanol wird mit 3,15 g einer 30-prozentigen Natriummethanolatlösung versetzt und 15 min bei Raumtemperatur gerührt. Die Reaktionsmischung wird vollständig eingeengt und der Rückstand wird chromatographiert. Das resultierende Öl wird in 100 ml Ether gelöst und durch Zugabe von etherischer Salzsäure wird das Hydrochlorid ausgefällt.
Man erhält 2,3 g (48%) 3-Butylmercapto-4-(3-pyridylmethyloxy)-furoxanhydrochlorid.
Fp.: 111 - 113°C

**Beispiel 31**

**4-tert.-Butyl-3-(phenylmercapto)-furoxan**

**a)** 3,3-Dimethyl-2-oxo-butanthiohydroxamsäure-S-phenylester
Zu einer Lösung von 12,2 g (89 mmol) Chlorisonitrosopinakolin (Liebigs Ann.Chem. 444 (1925) 113) in 200 ml Ether wird eine Lösung von 9,8 g (89 mmol) Thiophenol in 30 ml Ether und dann eine Lösung von 9,3 g (92 mmol) Triethylamin in 50 ml Ether zugetropft. Nach 2h wird der ausgefallene Niederschlag abgesaugt, das Filtrat wird eingeengt und durch Verrühren mit Petrolether zur Kristallisation gebracht.
Man erhält 12 g (56%) 3,3-Dimethyl-2-oxo-butanthiohydroxamsäure-S-phenylester.
Fp.: 110 - 111°C
**b)** 3,3-Dimethyl-2-hydroxyimino-butanthiohydroxamsäure-S-phenylester
Eine Mischung von 60 g (0,25 mol) 3,3-Dimethyl-2-oxobutanthiohydroxamsäure-S-phenylester, 52,7 g (0,76 mol) Hydroxylammoniumchlorid und 62,2 g (0,76 mol) Natriumacetat in 250 ml Wasser und 250 ml Ethanol wird 18h auf 80°C erhitzt. Man versetzt die Reaktionsmischung mit 1500 ml Wasser, rührt über Nacht und saugt das ausgefallene Produkt ab. Nach Umkristallisation aus Isopropanol erhält man 35 g (55%) 3,3-Dimethyl-2-hydroxyimino-butanthiohydroxamsaure-S-phenylester.
Fp.: 178 - 180°C
**c)** Zu einer Suspension von 33,0 g (0,13 mol) 3,3-Dimethyl-2-hydroxyiminobutanthiohydroxamsäure-S-phenylester in 400 ml Ether werden 12,0 g (0,13 mol) Distickstofftetroxid zugetropft. Nach 2h bei Raumtemperatur wird der Ansatz am Rotationsverdampfer eingeengt, und der Rückstand wird aus Isopropanol umkristallisiert.
Man erhält 24,0 g (73%) 4-tert.-Butyl-3-(phenylmercapto)-furoxan.
Fp.: 55°C

**Beispiel 32**

**4-tert.-Butyl-3-phenylsulfinylfuroxan**

Zu einer Lösung von 3,0 g (12 mmol) 4-tert.-Butyl-3-(phenylmercapto)-furoxan in 30 ml Trifluoressigsäure werden 4,7 g (48 mmol) einer 35%igen Wasserstoffperoxidlösung zugetropft. Nach genau 7 min wird die Reaktionsmischung auf 90 g Eiswasser gegossen und das ausgefallene Produkt wird abgesaugt. Nach Umkristallisation aus Ethanol/Wasser 3:2 werden 2,9 g (91%) 4-tert.-Butyl-3-phenylsulfinylfuroxan erhalten.
Fp.: 89 - 91°C

**Beispiel 33**

**4-tert.-Butyl-3-phenylsulfonylfuroxan**

Eine Lösung von 14,8 g (59 mmol) 4-tert.-Butyl-3-(phenylmercapto)-furoxan in 140 ml Trifluoressigsäure und 25 g (257 mmol) 35% Wasserstoffperoxid wird 24h bei Raumtemperatur gerührt. Man gießt auf 350 g Eiswasser, saugt den Niederschlag ab, kristallisiert aus Ethanol um und erhält 15,1 g (90%) 4-tert.-Butyl-3-phenylsulfonylfuroxan.
Fp.: 90 - 92°C

**Beispiel 34**

**4-tert.-Butyl-3-butylmercaptofuroxan**

Zu einer Suspension von 10,0 g (35 mmol) 4-tert.-Butyl-3-phenylsulfonylfuroxan in 150 ml Methanol werden nacheinander 3,7 g (41 mmol) Butylmercaptan und 7,4 g (41 mmol) 30%ige Natriummethanolatlösung zugegeben. Man rührt 3h bei Raumtemperatur unter Stickstoff und destilliert dann das Lösungsmittel ab. Der Rückstand wird in 300 ml Cyclohexan und 100 ml Wasser aufgenommen, die wäßrige Phase 1x mit Cyclohexan extrahiert und die vereinigten organischen Phasen werden eingeengt. Nach chromatographischer Reinigung erhält man 5,5 g (67%) 4-tert.-Butyl-3-butylmercaptofuroxan.

**Beispiel 35**

**4-tert.-Butyl-3-butylsulfinylfuroxan**

Aus 4-tert.-Butyl-3-butylmercaptofuroxan wurde analog Beispiel 32 4-tert.-Butyl-3-butylsulfinylfuroxan als Öl erhalten.
$^{13}C$-NMR: $\delta$ = 163,8 (s), 116,6 (s), 47,9 (t), 33,4 (s), 28,0 (q), 24,2 (t), 21,1 (t), 13,3 (q).

**Beispiel 36**

**4-tert.-Butyl-3-butylsulfonylfuroxan**

Aus 4-tert.-Butyl-3-butylmercaptofuroxan wurde analog Beispiel 33 4-tert.-Butyl-3-butylsulfonylfuroxan erhalten.
Fp.: 77 - 79°C

**Patentansprüche**

1. Furoxane der allgemeinen Formel I

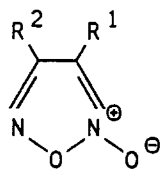

(I)

worin einer der Reste $R^1$ und $R^2$ für $-S(O)_n-R^3$ und der andere für $(C_1-C_{10})$-Alkyl, $(C_3-C_7)$-Cycloalkyl, $-CONR^4R^5$, -CN oder $-XR^6$ steht, wobei

n 0, 1 oder 2 bedeutet;

$R^3$ $(C_1-C_{10})$-Alkyl, Hydroxy-$(C_1-C_{10})$-alkyl, $R^7R^8N-(C_1-C_{10})$-alkyl, $(C_2-C_{22})$-Alkyl, das durch ein, zwei oder drei Sauerstoffatome unterbrochen ist, $(C_3-C_7)$-Cycloalkyl, $(C_7-C_{10})$- Aralkyl, $-(CH_2)_mCOY$, Pyridylmethyl,$(C_6-C_{14})$-Aryl 5- bis 14-gliedriges Heteroaryl, $(C_6-C_{14})$-Aryl oder 5- bis 14-gliedriges Heteroaryl, die ein oder mehrfach substituiert sind durch einen oder mehrere Gruppen aus der Reihe $(C_1-C_5)$-Alkyl, $(C_3-C_7)$-Cycloalkyl, Formyl, $(C_1-C_4)$-Alkylcarbonyl, Amino, $(C_1-C_5)$-Alkylamino, Di-$(C_1-C_5)$-alky-

lamino, Hydroxy, $(C_1-C_5)$-Alkoxy, Nitro, Cyano oder Halogen, oder $-CH_2CH_2SCOO-(C_1-C_4)$-Alkyl bedeutet;

$R^4$ und $R^5$ unabhängig voneinander Wasserstoff bedeuten oder wie $R^3$ definiert sind;

$R^6$ unabhängig von diesem wie $R^3$ definiert ist, wobei $-CH_2CH_2SCOO(C_1-C_4)$-Alkyl ausgeschlossen ist und wobei, falls X für Schwefel steht, $R^6$ zusammen mit $R^3$ eine Ethylengruppe bilden kann.

$R^7$ und $R^8$ unabhängig voneinander Wasserstoff, $(C_1-C_{10})$-Alkyl, $(C_7-C_{10})$-Aralkyl oder $(C_6-C_{14})$-Aryl, das wie in der Definition von $R^3$ angegeben substituiert sein kann, bedeuten;

X Sauerstoff oder Schwefel bedeutet;

Y Hydroxy, $(C_1-C_4)$-Alkoxy, Amino, $(C_1-C_4)$-Alkylamino oder Di-$(C_1-C_4)$-Alkylamino bedeutet; und

m 1, 2 oder 3 bedeutet;

sowie deren pharmakologisch annehmbare Salze, wobei, wenn einer der beiden Reste $R^1$ und $R^2$ für Methyl steht, $R^3$ nicht Phenyl oder durch Methyl, Methoxy, Chlor oder Fluor para-substituiertes Phenyl sein kann; wenn einer der beiden Reste $R^1$ und $R^2$ für Methyl steht und n = 0 oder 2 bedeutet, $R^3$ nicht Methyl oder Ethyl sein kann; wenn einer der beiden Reste $R^1$ und $R^2$ für Methyl steht und n=2 bedeutet, $R^3$ nicht Benzyl sein kann; und wenn einer der beiden Reste $R^1$ und $R^2$ für Ethoxy steht, der andere nicht $-SO_2C_6H_5$ oder $-SO_2C_6H_4-CH_3$ sein kann.

**2.** Furoxane gemäß Anspruch 1, dadurch gekennzeichnet, daß der Rest $-S(O)_nR^3$ $-S(O)_n-(C_1-C_4)$-Alkyl, $-S(O)_n$-Hydroxy-$(C_1-C_4)$-alkyl, $-S(O)_n$-Cyclohexyl, $-S(O)_nCH_2COY'$, worin Y' Hydroxy oder $(C_1-C_4)$-Alkoxy bedeutet, $-S(O)_n$-Phenyl oder $-S(O)_n-CH_2CH_2SCOO(C_1-C_4)$-alkyl bedeutet.

**3.** Furoxane gemäß Anspruch 1 und/oder 2, dadurch gekennzeichnet, daß derjenige der Reste $R^1$ und $R^2$, der nicht für $-S(O)_nR^3$ steht, $(C_1-C_4)$-Alkyl, $-CONHR^{4'}$, worin $R^{4'}$ Wasserstoff, $(C_1-C_4)$-Alkyl oder durch Halogen substituiertes Phenyl bedeutet, -CN, Cyclohexylthio, Phenylthio, $(C_1-C_4)$-Alkoxy, eine Gruppe der Formel

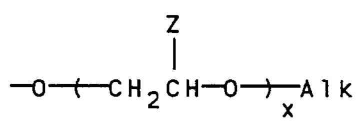

worin Z Wasserstoff oder Methyl, x 1, 2 oder 3 und Alk $(C_1-C_6)$-Alkyl bedeutet, Pyrid-3-yl-methyloxy, $R^{7'}R^{8'}-N-(C_1-C_4)$-alkoxy, worin $R^{7'}$ und $R^{8'}$ unabhängig voneinander Methyl oder Benzyl bedeuten, oder $SCH_2COY'$, worin Y' wie in Anspruch 2 angegeben, definiert ist, bedeutet.

**4.** Verfahren zur Herstellung von Furoxanen der allgemeinen Formel I gemäß einem oder mehreren der Ansprüche 1 bis 3, dadurch gekennzeichnet, daß eine Verbindung der allgemeinen Formel II

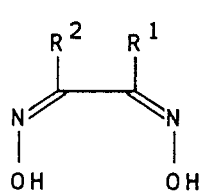

(II)

worin $R^1$ und $R^2$ wie in Anspruch 1 angegeben definiert sind, oxidiert wird.

**5.** Verwendung von Furoxanen der allgemeinen Formel I gemäß einem der Ansprüche 1 bis 3 zur Bekämpfung und Vorbeugung von Erkrankungen des kardiovaskulären Systems.

**6.** Verwendung von Furoxanen der allgemeinen Formel I gemäß einem der Ansprüche 1 bis 3 zur Behandlung erektiler Dysfunktionen.

7. Pharmazeutisches Präparat, dadurch gekennzeichnet, daß es ein Furoxan der allgemeinen Formel I gemäß einem der Ansprüche 1 bis 3, oder ein pharmakologisch annehmbares Salz davon als Wirkstoff zusammen mit pharmazeutisch annehmbaren Träger- und Zusatzstoffen und gegebenenfalls noch ein oder mehrere andere pharmakologische Wirkstoffe enthält.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 95 10 6885

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| A,D | WO-A-94 01422 (CHIESI FARMACEUTICI SPA) * Tabelle 2, Seiten 10-12; Ansprüche * --- | 1,5-7 | C07D271/08 A61K31/41 C07D413/12 C07D498/04 |
| A,D | EP-A-0 571 795 (CASSELLA AG) * Seite 4, Zeile 47 - Seite 5, Zeile 7; Ansprüche * --- | 1,4-7 | |
| A,D | J. MED. CHEM., Bd. 35, Nr. 17, 1992 Seiten 3296-3300, R. CALVINO ET AL * Schema I; Tabelle I, Verbindungen 3, 4, 6, 7, 9, 10; Tabelle III, Verbindungen 3a, 4a, 6a, 7a, 9a-b, 10a-b * --- -/-- | 1,4,5,7 | |

RECHERCHIERTE SACHGEBIETE (Int.Cl.6)

C07D
A61K

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| BERLIN | 23.Juni 1995 | Van Amsterdam, L |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 95 10 6885

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| A | CHEMICAL ABSTRACTS, vol. 105, no. 13, 1986<br>Columbus, Ohio, US;<br>abstract no. 114403a,<br>* Zusammenfassung *<br>& DATABASE REGISTRY<br>CHEMICAL ABSTRACTS SERVICE, COLUMBUS, OHIO, US<br>STN,<br>*RN: 104151-75-9, 104151-74-8; 104151-88-4; 104151-87-3 *<br>& KHIM. GETEROSIKL. SOEDIN, Nr. 2, 1986<br>Seiten 264-266,<br>V.G. ANDRIANOV ET AL<br>--- | 1 | |
| A | J. CHEM. SOC. PERKIN TRANS. II, Nr. 12, 1973<br>Seiten 1613-1617,<br>A. GASCO ET AL<br>* Tabelle 1, Verbindungen 11, 13 *<br>--- | 1 | RECHERCHIERTE SACHGEBIETE (Int.Cl.6) |
| A,D | HETEROCYCLES,<br>Bd. 24, Nr. 4, 1986<br>Seiten 889-892,<br>T. SHIMIZU ET AL<br>* Seite 891, Verbindungen 6 *<br>--- | 1 | |
| A,D | J. CHEM. SOC.,<br>1964<br>Seiten 904-906,<br>W.V. FARRAR<br>* Seite 906, Zeile 18 - Zeile 25 *<br>----- | 1 | |

EP 95 10 6885

-C-

Bemerkung: Obwohl die Ansprüche 5,6 sich auf ein Verfahren zur Behandlung des menschlichen/tierischen Körpers (Diagnostizierverfahren, das am menschlichen/ tierischen Körper vorgenommen wird,) beziehen (Art. 52(4)EPU), wurde die Recherche durchgeführt und gründete sich auf die angeführten Wirkungen der Verbindung/ Zusammensetzung.